(19) **Europäisches Patentamt**

**European Patent Office**

**Office européen des brevets**

(11) Publication number: **0 540 676 B1**

(12) **EUROPEAN PATENT SPECIFICATION**

(45) Date of publication of patent specification: **17.11.94** (51) Int. Cl.⁵: **C07K 7/06, A61K 37/43**

(21) Application number: **91915321.3**

(22) Date of filing: **23.07.91**

(86) International application number:
**PCT/US91/05208**

(87) International publication number:
**WO 92/01711 (06.02.92 92/04)**

(54) **PEPTIDE COMPOUNDS HAVING GROWTH HORMONE RELEASING ACTIVITY.**

(30) Priority: **24.07.90 US 557226**
**24.07.90 US 558120**

(43) Date of publication of application:
**12.05.93 Bulletin 93/19**

(45) Publication of the grant of the patent:
**17.11.94 Bulletin 94/46**

�ividedDesignated Contracting States:
**AT BE CH DE DK ES FR GB GR IT LI LU NL SE**

(56) References cited:
**EP-A- 0 018 072        WO-A-87/06835**
**WO-A-88/09780        WO-A-89/07110**
**WO-A-89/07111        WO-A-89/10933**

(73) Proprietor: **POLYGEN HOLDING CORPORA-TION**
**1201 North Market Street**
**Wilmington, Delaware 19899 (US)**

(72) Inventor: **BOWERS, Cyril, Y.**
**484 Audobon Street**
**New Orleans, LA 70118 (US)**

Inventor: **HUBBS, John, C.**
**Route 10, Box 354**
**Kingsport, TN 37664 (US)**
Inventor: **FOSTER, Charles, H.**
**1413 Dobyns Drive**
**Kingsport, TN 37664 (US)**
Inventor: **CODY, Wayne, L.**
**1314 Maplewood Drive**
**Saline, MI 48176 (US)**
Inventor: **MOMANY, Frank, A.**
**611 Washington Street 202**
**Wellesley, MA 02181 (US)**

(74) Representative: **Davies, Jonathan Mark**
**Reddie & Grose**
**16 Theobalds Road**
**London WC1X 8PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

This invention relates to novel polypeptide compounds which promote the release of growth hormone when administered to animals. In another aspect, this invention relates to methods for promoting the release and elevation of growth hormone levels in animals by administration of specified growth hormone releasing polypeptide compounds thereto.

Background Of The Invention

The elevation of growth hormone (GH) levels in mammals upon administration of GH-releasing compounds can lead to enhanced body weight and to enhanced milk production if sufficiently elevated GH levels occur upon administration. Further, it is known that the elevation of growth hormone levels in mammals can be accomplished by application of known growth hormone releasing agents, such as the naturally occurring growth hormone releasing hormones.

The elevation of growth hormone levels in mammals can also be accomplished by application of growth hormone releasing peptides, some of which have been previously described, for example, in U.S. 4,223,019, U.S. 4,223,020, U.S. 4,223,021, U.S. 4,224,316, U.S. 4,226,857, U.S. 4,228,155, U.S. 4,228,156, U.S. 4,228,157, U.S. 4,228,158, U.S. 4,410,512, U.S. 4,410,513, U.S. 4,411,890 and U.S. 4,839,344.

Antibodies to the endogenous growth hormone release inhibitor, somatostatin (SRIF) have also been used to cause elevated GH levels. In this latter example, growth hormone levels are elevated by removing the endogenous GH-release inhibitor (SRIF) before it reaches the pituitary, where it inhibits the release of GH.

Each of these methods for promoting the elevation of growth hormone levels involve materials which are expensive to synthesize and/or isolate in sufficient purity for administration to a target animal. Short chain, relatively simple polypeptides which have the ability to promote the release of growth hormone would be desirable because they should be readily and inexpensively prepared, easily modified chemically and/or physically, as well as easily purified and formulated; and they should have excellent transport properties.

It would be desirable to have short chain polypeptides which promote the release and elevation of growth hormone levels in the blood of animals. It would also be useful to be able to use such polypeptides to promote the release and elevation of growth hormone levels in the blood of animals.

Prior art documents WO 89/07110, WO 89/07111 and WO 89/10933 described short chain polypeptides which have GH releasing activity. These documents show polypeptides which respectively have the general formula: X-AA2-AA3-Trp-AA5-Y-Z, AA1-AA2-AA3-Trp-AA5-AA6-AA7-Z and Ala-AA2-AA3-Trp-AA5-Y-Z. DPhe and DTrp is suggested in the AA2 position in each case.

Summary Of Invention

We have now discovered several novel polypeptide compounds which promote the release of growth hormone in animals. These polypeptides have the formula $A_1$-$A_2$-$A_3$-Trp-$A_4$-$A_5$-Z wherein $A_1$ is His, 3(NMe)-His, $A_0$-His, $A_0$-3(NMe)His, Ala, Tyr or His-Ala, wherein $A_0$ is any naturally occurring L-amino acid, Met(0), DOPA, Abu, or peptides of the formula, L-$A_0$, wherein L is H, DOPA, Lys, Phe, Tyr, Cys, Tyr-DAla-Phe-Gly, Tyr-DAla-Gly-Phe, Tyr-Ala-Gly-Thr or Tyr-DAla-Phe-Sar, preferably $A_0$ is any naturally occurring amino acid, more preferably, $A_0$ is Ala, Lys, or Glu; $A_2$ is $D^\beta$Nal; $A_3$ is Ala, Gly or Ser; $A_5$ is DPhe, D/L$^\beta$(Me)Phe or (NMe)DPhe; $A_5$ is B-G or G, wherein B is any naturally-occurring amino acid, dipeptides of any naturally-occurring amino acids or $H_2N$-$(CH_2)_n$-$CO_2H$ (wherein n = 2-12) and G is Arg, iLys, or Orn; Z is the C terminal end group -$CONR^1R^2$, -$COOR^1$ or -$CH_2OR^1$ (wherein $R^1$ is H, an alkyl group having from 1 to about 6 carbon atoms, a cycloalkyl group having from 3 to about 8 carbon atoms, an alkenyl group having from 2 to about 8 carbon atoms or an aryl group having from 6 to about 12 carbon atoms, and $R^2$ is defined as $R^1$ and may be the same or different), -Gly-Z', -Met-Z', -Lys-Z', -Cys-Z', -Gly-Tyr-Z', or -Ala-Tyr-Z', wherein Z' is -$CONR^1R^2$, -$COOR^1$ or -$CH_2OR^1$ (wherein $R^1$ and $R^2$ are as defined above), and the organic or inorganic pharmaceutically acceptable salts thereof. Preferably, the peptide has the formula

His-$A_2$-Ala-Trp-DPhe-Lys-$NH_2$,
$A_0$-His-$A_2$-Ala-Trp-DPhe-Lys-$NH_2$ (for example
Ala-His-$A_2$-Ala-Trp-DPhe-Lys-$NH_2$) or
Ala-$A_2$-Ala-Trp-DPhe-Lys-$NH_2$.

2

Such peptides can be used to promote the release and elevation of blood growth hormone levels in animals by administering an effective amount of the peptide.

Detailed Description Of The Invention

The present invention is based on the discovery of several short chain polypeptides which promote the release and elevation of growth hormone levels in the blood of animals. The polypeptides contemplated to be within the scope of the present invention are defined by the following generic structure:

$A_1$-$A_2$-$A_3$-Trp-$A_5$-$A_5$-Z, wherein $A_1$, $A_2$ $A_3$, $A_5$, $A_5$ and Z are as defined below. $A_1$ is His, 3(NMe)His (i.e., wherein the imidazole ring is methylated at the 3-position), His-Ala, $A_0$-His, Ala, Tyr or $A_0$-3(NMe)His, where $A_0$ is any naturally occurring L-amino acid, Met(O), DOPA, Abu or peptides of the formula, L-$A_0$, wherein L is H. DOPA, Lys, Phe, Tyr, Cys, Tyr-DAla-Phe-Gly, Tyr-DAla-Gly-Phe, Tyr-Ala-Gly-Thr or Tyr-DAla-Phe-Sar. Preferably, $A_1$ is His, Ala, His-Ala, $A_0$-His, or Ala. More preferably, $A_1$ is His or $A_0$-His. $A_0$ is preferably any naturally ocurring L-amino acid, more preferably, $A_0$ is Ala, Lys or Glu. Still more preferably, $A_0$ is Ala.

$A_2$ is D$^\beta$Nal.

$A_3$ is Ala, Gly or Ser. $A_3$ is preferably Ala.

$A_5$ is DPhe D/L$^\beta$(Me)Phe or (NMe)DPhe. Preferably, $A_5$ is DPhe.

$A_5$ is B-G or G, wherein B is any naturally occurring L-amino acid, dipeptides of any naturally occurring L-amino acids (such as Ala-Lys, Ala-Ala, Ala-Leu) or $H_2N(CH_2)_nCO_2H$, wherein n = 2-12, and G is Arg, iLys, Lys or Orn. More preferably B is any naturally ocurring L-amino acid or $H_2N(CH_2)_n CO_2H$, wherein n = 4-8. Still more preferably, $A_5$ is G, Ala-Lys, or Ala-Ala-Lys. Even more preferably, $A_5$ is G. Most preferably, $A_5$ is Lys.

Z represents the C terminal end group of the polypeptide or the C terminal amino acid(s) plus end group, wherein Z is -CONR$^1$R$^2$, -COOR$^1$ or -CH$_2$OR$^1$, wherein R$^1$ is H, an alkyl group having from 1 to about 6 carbon atoms, a cycloalkyl group having from 3 to about 8 carbon atoms, an alkenyl group having from 2 to about 8 carbon atoms, or an aryl group having from 6 to about 12 carbon atoms. R$^2$ is defined as R$^1$ and may be the same or different. Preferably, R$^1$ is H or an alkyl group having from 1 to about 6 carbon atoms. Z can also be -Gly-Z', -Met-Z', -Lys-Z', -Cys-Z', -Gly-Tyr-Z', or -Ala-Tyr-Z', wherein Z' is -CONR$^1$R$^2$, -COOR$^1$ or -CH$_2$OR$^1$, wherein R$^1$ and R$^2$ are as defined above. Preferably, Z is -CONR$^1$R$^2$, -COOR$^1$ or -CH$_2$OR$^1$.

And the pharmaceutically acceptable organic or inorganic addition salts of any of said polypeptides.

The amino acid residue abbreviations used are in accordance with the standard peptide nomenclature:

Gly = Glycine
Tyr = L-Tyrosine
Ile = L-Isoleucine
Glu = L-Glutamic Acid
Thr = L-Threonine
Phe = L-Phenylalanine
Ala = L-Alanine
Lys = L-Lysine
Asp = L-Aspartic Acid
Cys = L-Cysteine
Arg = L-Arginine
Gln = L-Glutamine
Pro = L-Proline
Leu = L-Leucine
Met = L-Methionine
Ser = L-Serine
Asn = L-Asparagine
His = L-Histidine
Trp = L-Tryptophan
Val = L-Valine
DOPA = 3,4-Dihydroxyphenylalanine
Met(O) = Methionine Sulfoxide
Abu = α-Aminobutyric Acid
iLys = N$^\epsilon$-Isopropyl-L-Lysine
4-Abu = 4-Aminobutyric Acid
Orn = L-Ornithine

$D^{\alpha}Nal$ = $\alpha$-Naphthyl-D-Alanine
$D^{\beta}Nal$ = $\beta$-Naphthyl-D-Alanine
Sar = Sarcosine

All three letter amino acid abbreviations preceded by a "D" indicated the D-configuration of the amino acid residue, and abbreviations preceded by a "D/L" indicate a mixture of the D- and L-configurations of the designated amino acid. For purposes of this disclosure, glycine is considered to be included in the term "naturally occurring L-amino acids".

The basic, neutral or acidic amino acid residues that can be used for amino acids $A_1$, $A_3$, $A_5$ and $A_5$ provide one with a great deal of control over the physiochemical properties of the desired peptide. Such flexibility provides important advantages for the formulation and delivery of the desired peptide to any given species. Additional flexibility is gained by the selection of R and Z moieties, as well, thereby providing added control over the physiochemical properties of the desired compound.

Preferred growth hormone releasing compounds employed in the practice of the present invention are:

$A_1$-$A_2$-Ala-Trp-DPhe-$A_5$-Z, such as

$A_0$-His-$A_2$-Ala-Trp-DPhe-$A_5$-Z,

His-Ala-$A_2$-Ala-Trp-DPhe-$A_5$-Z,

Ala-$A_2$-Ala-Trp-DPhe-$A_5$-Z and

His-$A_2$-Ala-Trp-DPhe-$A_5$-Z, and

organic or inorganic addition salts thereof.

Preferable are

$A_0$-His-$D^{\beta}Nal$-Ala-Trp-DPhe-Lys-$NH_2$ (particularly,

Ala-His-$D^{\beta}Nal$-Ala-Trp-DPhe-Lys-$NH_2$), and

His-$D^{\beta}Nal$-Ala-Trp-DPhe-Lys-$NH_2$, and

organic or inorganic addition salts of any of said polypeptides.

These compounds are typically easy to synthesize, have efficacy at promoting an increase in serum growth hormone levels, and are desirable for commercial scale production and utilization. In addition, these compounds may be advantageous in having physiochemical properties which are desirable for the efficient delivery of such polypeptide compounds to a wide variety of animal species because of the flexibility made possible by the various substitutions at numerous positions of the polypeptide compounds, by selecting the polar, neutral or non-polar nature of the N-terminal, C-terminal and center portions of these polypeptide compounds so as to be compatible with the desired method of delivery.

These peptides typically show a higher level of potency at promoting the increase in serum growth hormone levels than most equivalent peptides with a different amino acid residue at the $A_2$ position.

His-$D^{\beta}Nal$-Ala-Trp-DPhe-Lys-$NH_2$ and

$A_0$-His-$D^{\beta}Nal$-Ala-Trp-DPhe-Lys-$NH_2$ peptides (such as Ala-His-$D^{\beta}Nal$-Ala-Trp-DPhe-Lys-$NH_2$),

and organic or inorganic addition salts thereof are presently the most preferred.

These compounds have been shown to have a high level of potency at promoting the increase in serum growth hormone levels.

The compounds of this invention may be used to enhance blood GH levels in animals; enhance milk production in cows; enhance body growth in animals such as mammals (e.g., humans, sheep, bovines, and swine), as well as fish, fowl, other vertebrates and crustaceans; and increase wool and/or fur production in mammals. The amount of body growth is dependent upon the sex and age of the animal species, quantity and identity of the growth hormone releasing compound being administered, route of administration, and the like.

The novel polypeptide compounds of this invention can be synthesized according to the usual methods of solution and solid phase peptide chemistry, or by classical methods known in the art. The solid-phase synthesis is commenced from the C-terminal end of the peptide. A suitable starting material can be prepared, for instance, by attaching the required protected alpha-amino acid to a chloromethylated resin, a hydroxymethyl resin, a benzhydrylamine (BHA) resin, or a para-methyl-benzylhydrylamine (p-Me-BHA) resin. One such chloromethyl resin is sold under the tradename BIOBEADS SX-1 by Bio Rad Laboratories, Richmond, California. The preparation of the hydroxymethyl resin is described by Bodansky et al., Chem. Ind. (London) 38, 1597 (1966). The BHA resin has been described by Pietta and Marshall, Chem. Comm., 650 (1970) and is commercially available from Peninsula Laboratories, Inc., Belmont, California.

After the initial attachment, the alpha-amino protecting group can be removed by a choice of acidic reagents, including trifluoroacetic acid (TFA) or hydrochloric acid (HCl) solutions in organic solvents at room temperature. After removal of the alpha-amino protecting group, the remaining protected amino acids can be coupled stepwise in the desired order. Each protected amino acid can be generally reacted in about a 3-fold excess using an appropriate carboxyl group activator such as dicyclohexylcarbodiimide (DCC) or diisopropyl carbodiimide (DIC) in solution, for example, in methylene chloride ($CH_2Cl_2$) or dimethylformamide (DMF) and mixtures thereof.

After the desired amino acid sequence has been completed, the desired peptide can be cleaved from the resin support by treatment with a reagent such as hydrogen fluoride (HF) which not only cleaves the peptide from the resin, but also cleaves most commonly used side-chain protecting groups. When a chloromethyl resin or hydroxymethyl resin is used, HF treatment results in the formation of the free peptide acid. When the BHA or p-Me-BHA resin is used, HF treatment results directly in free peptide amides.

The solid-phase procedure discussed above is well known in the art and has been described by Stewart and Young, Solid Phase Peptide Synthesis: Second Edn. (Pierce Chemical Co., Rockford, IL 1984).

Some solution methods which can be employed to synthesize the peptide moieties of the instant invention are set forth in Bodansky et al ., Peptide Synthesis, 2nd Edition, John Wiley & Sons, New York, N.Y. 1976.

It is believed that the peptides will be more preferably synthesized by a soluton phase method which involves the condensation reaction of at least two peptide fragments.

This method comprises condensing a peptide fragment X-$A_1$-Y with the peptide fragment U-V-W, wherein all amino acid side-chains except for $A_1$ are neutral or protected, and wherein X is Prot. or Prot . -$A_0$, where Prot. is an N-terminus protecting group; Y is $A_2$-Q, Ala-$A_2$-Q, $A_2$-$A_3$-Q, Ala-$A_2$-$A_3$-Q, $A_2$-$A_3$-$A_4$-Q, Ala-$A_2$-$A_3$-$A_4$-Q, Ala-Q or -Q, where when Y is Q, U is J-$A_2$-$A_3$-$A_4$, or J-Ala-$A_2$-$A_3$-$A_4$. When Y is Ala-Q, U is J-$A_2$-$A_3$-$A_4$. When Y is $A_2$-Q or Ala-$A_2$-Q, U is J-$A_3$-$A_4$. When Y is $A_2$-$A_3$-Q or Ala-$A_2$-$A_3$-Q, U is J-$A_4$. When Y is $A_2$-$A_3$-$A_4$-Q or Ala-$A_2$-$A_3$-$A_4$-Q, U is J. V is $A_5$ or Z. When V is $A_5$, W is $A_5$-Z or Z. When V is Z, W is not present. $A_1$, $A_5$, $A_5$ and Z are as defined herein. In the present situation $A_2$ is $D^\beta$Nal and $A_4$ is Trp.

Q is the carboxy terminus of a peptide fragment and is -$OR^3$ or -M, where M is a moiety capable of being displaced by a nitrogen-containing nucleophile and $R^3$ is H, an alkyl group containing one to about 10 carbon atoms, an aryl group having from 6 to about 12 carbon atoms or an arylalkyl group having from 7 to about 12 carbon atoms; J represents the amine terminus of the indicated fragment and is H or a protecting group, which does not hinder the coupling reaction, for example, benzyl.

Thereafter, one removes the protecting groups. Alternatively, one may use the protected peptide thus formed in further condensations to prepare a larger peptide.

This preferred method is more fully described in U.S. Patent Application Serial Number        filed on July 24, 1990, by John C. Hubbs and S.W. Parker entitled "Process for Synthesizing Peptides", which is incorporated herein by reference.

In accordance with another embodiment of the present invention, a method is provided for promoting release and/or elevation of growth hormone levels in the blood of a non-human animal. Said method comprises administering to a non-human animal an effective dose of at least one of the above-described polypeptides.

The compounds of this invention can be administered by oral, parenteral (intramuscular (i.m.), intraperitoneal (i.p.), intravenous (i.v.) or subcutaneous (s.c.) injection), nasal, vaginal, rectal or sublingual routes of administration and can be formulated in dose forms appropriate for each route of administration. Parenteral administration is preferred.

Solid dose forms for oral administration include capsules, tablets, pills, powders and granules. In such solid dose forms, the active compound is mixed with at least one inert carrier such as sucrose, lactose, or starch. Such dose forms can also comprise, as is normal practice, additional substances other than inert diluents, e.g., lubricating agents such as magnesium stearate. In the case of capsules, tablets and pills, the dose forms may also comprise buffering agents. Tablets and pills can additionally be prepared with enteric coatings.

Liquid dose forms for oral administration include emulsions, solutions, suspensions, syrups, the elixirs containing inert diluents commonly used in the art, such as water. Besides, such inert diluents, compositions can also include adjuvants, such as wetting agents, emulsifying and suspending agents, and sweetening, flavoring, and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dose forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in a medium of sterile water, or some other sterile injectable medium immediately before use.

The novel compounds of the present invention are also useful when administered in combination with growth hormone releasing hormone (i.e., naturally occurring growth hormone releasing hormone, analogs and functional equivalents thereof), as well as in combination with other compounds which promote the release of growth hormone, e.g., growth hormone releasing peptides (see U.S. Patent No. 4,880,778 which is incorporated herein by reference). Such combinations represent an especially preferred means to administer the growth hormone releasing peptides of the present invention because the combination promotes the release of much more growth hormone than is predicted by the summation of the individual responses for each component of the combination, i.e., the combination provides a synergistic response relative to the individual component. Further details on the administration of combinations of growth hormone releasing peptides are described in the above-cited patent. Such synergistic compounds are preferably compounds which act as an agonist at the growth hormone releasing hormone receptor or inhibit the effect of somatostatin. The synergism can be binary, i.e. the present compound and one of the synergistic compounds, or involve more than one synergistic compound.

The amount of polypeptide or combination of polypeptides of the present invention administered will vary depending on numerous factors, e.g., the particular animal treated, its age and sex, the desired therapeutic affect, the route of administration and which polypeptide or combination of polypeptides are employed. In all instances, however, a dose effective to promote release and elevation of growth hormone level in the blood of the recipient animal is used. Ordinarily, this dose level falls in the range of between about 0.1 $\mu$g to 10 mg of total polypeptide per kg of body weight. The preferred amount can readily be determined empirically by the skilled artisan based upon the present disclosure.

For example, in humans when the mode of administration is i.v. the preferred dose level falls in the range of about 0.1 $\mu$g to 10 $\mu$g of total polypeptide per kg of body weight, more preferably, about 0.5 $\mu$g to 5 $\mu$g of total polypeptide per kg of body weight, still more preferably about .7 $\mu$g about 3.0 $\mu$g per kg of body weight. When combinations of growth hormone releasing peptides are used, lower amounts of the presently described peptide can be used. For example, combining the presently described peptide with, for example, a synergistic compound in Group I of U.S. Patent No. 4,880,778 such as GHRH, a preferred range is about 0.1 $\mu$g to about 5 $\mu$g of the presently described compound (e.g.
Ala-His-D$^\beta$Nal-Ala-Trp-DPhe-A$_6$-Z,
or His-D$^\beta$Nal-Ala-Trp-DPhe-A$_6$-Z) per kg of body weight and about .5 $\mu$g to about 15.0 $\mu$g of synergistic compound (e.g. GHRH) and more preferably about 0.1 $\mu$g to about 3 $\mu$g of the present compound with about 1.0 $\mu$g to about 3.0 $\mu$g of the synergistic compound per kg of body weight.

When the mode of administration is oral, greater amounts are typically needed. For example, in humans for oral administration, the dose level is typically about 30 $\mu$g to about 600 $\mu$g of polypeptide per kg of body weight, more preferably about 70 $\mu$g to about 500 $\mu$g of polypeptide per kg of body weight, still more preferably, about 100 $\mu$g to about 350 $\mu$g of total polypeptide per kg of body weight, even more preferably, about 200 $\mu$g to about 300 $\mu$g of total polypeptide per kg of body weight. Cows require about the same dose level as humans, while rats typically require higher dose levels. The exact level can readily be determined empirically based upon the present disclosure.

In general, as aforesaid, the administration of combinations of growth hormone releasing peptides will allow for lower doses of the individual growth hormone releasing compounds to be employed relative to the dose levels required for individual growth hormone releasing compounds in order to obtain a similar response, due to the synergistic effect of the combination.

Also included within the scope of the present invention are compositions comprising, as an active ingredient, the organic and inorganic addition salts of the above-described polypeptides and combinations thereof; optionally, in association with a carrier, diluent, slow release matrix, or coating.

The organic or inorganic addition salts of the growth hormone releasing compounds and combinations thereof contemplated to be within the scope of the present invention include salts of such organic moieties as acetate, trifluoroacetate, oxalate, valerate, oleate, laurate, benzoate, lactate, tosylate, citrate, maleate, fumarate, succinate, tartrate, naphthalate, and the like; and such inorganic moieties as Group I (i.e., alkali metal salts), Group II (i.e. alkaline earth metal salts) ammonium and protamine salts, zinc, iron, and the like with counterions such as chloride, bromide, sulfate, phosphate and the like, as well as the organic moieties referred to above.

Pharmaceutically acceptable salts are preferred when administration to human subjects is contemplated. Such salts include the non-toxic alkali metal, alkaline earth metal and ammonium salts commonly used in the pharmaceutical industry including sodium, potassium, lithium, calcium, magnesium, barium, ammonium and protamine salts which are prepared by methods well known in the art. The term also includes non-toxic acid addition salts which are generally prepared by reacting the compounds of this invention with a suitable organic or inorganic acid. Representative salts include hydrochloride, hydrobromide, sulfate, bisulfate, acetate, oxalate, valerate, oleate, laurate, borate, benzoate, lactate, phosphate, tosylate, citrate, maleate, fumarate, succinate, tartrate, napthylate, and the like.

The invention will be further illustrated by the following non-limiting examples.

Example 1

Synthesis of The Growth Hormone Releasing Peptides

Paramethyl benzhydrylamine hydrochloride (pMe-BHA•HCl) resin is placed in a reaction vessel on a commercially available automated peptide synthesizer. The resin is substituted with free amine up to a loading of about 5 mmoles per gram. The compounds are prepared by coupling individual amino acids starting at the carboxy terminus of the peptide sequence using an appropriate activing agent, such as N,N'-dicyclohexylcarbodiimide (DCC). The alpha amine of individual amino acids are protected, for example, as the t-butyloxycarbonyl derivative (t-Boc) and the reactive side chain functionalities are protected as outlined in Table 1.

Table 1

| Side Chain Protecting Groups Suitable For Solid Phase Peptide Synthesis | |
|---|---|
| Arginine: | $N^g$-Tosyl |
| Aspartic Acid: | O-Benzyl |
| Cysteine: | S-para-Methylbenzyl |
| Glutamic Acid: | O-Benzyl |
| Histidine: | $N^{im}$-Tosyl |
| Lysine: | $N^\epsilon$-2,4-Dichlorobenzyloxycarbonyl |
| Methionine: | S-Sulfoxide |
| Serine: | O-Benzyl |
| Threonine: | O-Benzyl |
| Tryptophan: | $N^{in}$-Formyl |
| Tyrosine: | O-2,6-Dichlorobenzyl |

Prior to incorporation of the initial amino acid, the resin is agitated three times (about one minute each) with dichloromethane ($CH_2Cl_2$; about 10 mL/gm of resin), neutralized with three agitations (about two minutes each) of N,N-diisopropylethylamine (DIEA) in dichloromethane (10:90; about 10 mL/gm of resin) and agitated three times (about one minute each) with dichloromethane (about 10 mL/gm of resin). The initial and each of the subsequent amino acids are coupled to the resin using a preformed symmetrical anhydride using about 3.0 times the total amount of the binding capacity of the resin of a suitably protected amino acid and about 1.5 times the total amount of the binding capacity of the resin of DCC in an appropriate amount of dichloromethane. For amino acids with a low dichloromethane solubility, N,N-dimethylformamide (DMF) is added to achieve a homogenous solution. Generally, the symmetrical anhydride is prepared up to 30 minutes prior to introduction into the reaction vessel at room temperature or below. The dicyclohexylurea that forms upon preparation of the symmetrical anhydride is removed via gravity filtration of the solution into the reaction vessel. Progress of the coupling of the amino acid to the resin is commonly monitored via a color test using a reagent such as ninhydrin (which reacts with primary and secondary amines). Upon complete coupling of the protected amino acid to the resin (>99%), the alpha amine protecting group is removed by treatment with acidic reagent(s). A commonly used reagent consists of a solution of trifluoroacetic acid (TFA), and anisole in dichloromethane (45:2:53). The complete procedure for incorporation of each individual amino acid residue onto the resin is outlined in Table 2.

Table 2

| Procedure for Incorporation Of Individual Amino Acids Onto a Resin | | |
|---|---|---|
| Reagent | Agitations | Time/Agitation |
| 1. Dichloromethane | 3 | 1 min. |
| 2. TFA, Anisole, Dichloromethane (45:2:53) | 1 | 2 min. |
| 3. TFA, Anisole, Dichloromethane (45:2:53) | 1 | 20 min. |
| 4. Dichloromethane | 3 | 1 min. |
| 5. DIEA, Dichloromethane (10:90) | 3 | 2 min. |
| 6. Dichloromethane | 3 | 1 min. |
| 7. Preformed symmetrical anhydride | 1 | 15-120 min.* |
| 8. Dichloromethane | 3 | 1 min. |
| 9. iso-Propanol | 3 | 1 min. |
| 10. Dichloromethane | 3 | 1 min. |
| 11. Monitor progress of the coupling reaction** | | |
| 12. Repeat steps 1-12 for each individual amino acid | | |

\* Coupling time depends upon the individual amino acid.

\** The extent of coupling can be generally monitored by a color test. If the coupling is incomplete, the same amino acid can be recoupled by repeating steps 7-11. If the coupling is complete the next amino acid can be coupled.

By employing this method of peptide synthesis, novel resin-bound polypeptides such as:

$A_1$-$D^\beta$Nal-$A_3$-Trp-$A_5$-$A_5$- R

are obtained (wherein $A_1$, $A_3$, $A_5$, and $A_5$ are as defined above, and R is a polymeric resin and the functional groups of the constituent amino acids are protected with suitable protecting groups as needed). Specific sequences (in appropriately protected form) which have been prepared include:

His-$D^\beta$Nal-Ala-Trp-DPhe-Lys- R ,

Ala-His-$D^\beta$Nal-Ala-Trp-DPhe-Lys- R ,

Ala-His-DTrp-Ala-Trp-DPhe-Lys- R ,

His-Ala-DTrp-Ala-Trp-DPhe-Lys- R ,

His-$D^\alpha$Nal-Ala-Trp-DPhe-Lys- R ,

His-DAsp-Ala-Trp-DPhe-Lys- R ,

His-DCys(SMe)-Ala-Trp-DPhe-Lys- R ,

His-DTrp-Ala-Trp-DPhe-Ala-Lys- R ,

His-$D^\beta$Nal-Ala-Trp-DPhe-Ala-Lys- R ,

Ala-His-$D^\beta$Nal-Ala-Trp-DPhe-Ala-Lys- R ,

Try-DArg-Phe-Gly- R,

Ala-His-Dphe-Ala-Trp-DPhe-Lys- R

Ala-His-DHis-Ala-Trp-DPhe-Lys -R, and

Ala-His-DPro-Ala-Trp-DPhe-Lys- R

## Example 2

### In Vivo GH Release In Rats

Immature female Sprague-Dawley rats were obtained from the Charles River Laboratories (Wilmington, MA). After arrival they were housed at 25°C with a 14:10 hour light:dark cycle. Water and Purina rat chow were available ad libitum. Pups were kept with their mothers until 21 days of age.

Twenty-six day old rats, six rats per treatment group, were anesthetized interperitoneally with 50 mg/Kg of pentobarbital 20 minutes prior to i.v. treatment with peptide. Normal saline with 0.1% gelatin was the vehicle for intravenous (i.v.) injections of the peptides. The anesthetized rats, weighing 55-65 grams, were injected i.v. with the quantity of growth hormone releasing compounds indicated in Tables 3, 4 and 5. Injection was made as a 0.2 mL solution into the jugular vein.

All animals were sacrificed by guillotine 10 minutes after the final test injection (see Tables 3 and 4). Trunk blood for the determination of blood GH levels was collected following decapitation. After allowing the blood to clot, it was centrifuged and the serum was separated from the clot. Serum was kept fozen until the day of sampling for radioimmunasay (RIA) determination of growth hormone levels according to the following procedure, as developed by the National Institute of Arthritis, Diabetes and Digestive and Kidney Diseases (NIADDK).

Reagents are generally added to the RIA analysis tubes at a single sitting, at refrigerator temperature (about 4°C) in the following sequence:

(a) buffer,
(b) "cold" (i.e., non-radioactive) standard or unknown serum sample to be analyzed,
(c) radio-iodinated growth hormone antigen, and
(d) growth hormone antiserum.

Reagent addition is generally carried out so that there is achieved a final RIA tube dilution of about 1:30,000 (antiserum to total liquid volume; vol:vol).

The mixed reagents are then typically incubated at room temperature (about 25°C) for about 24 hours prior to addition of a second antibody (e.g., goat or rabbit anti-monkey gamma globulin serum) which binds to and causes precipitation of the complexed growth hormone anti serum. Precipitated contents of the RIA tubes are then analyzed for the number of counts in a specified period of time in a gamma scintillation counter. A standard curve is prepared by plotting number of radioactive counts versus growth hormone (GH) level. GH levels of unknowns are then determined by reference to the standard curve.

Serum GH was measured by RIA with reagents provided by the National Hormone and Pituitary Program.

Serum levels in Tables 3, 4, 5 and 6 are recorded in ng/mL in terms of the rat GH standard of 0.61 International Units/mg (IU/mg). Data is recorded as the mean +/- standard error of the mean (SEM). Statistical analysis was performed with Student's t-test. NS means the difference was not statistically significant. In Tables 3, 4, 5 and 6 the results shown are the average of studies with six rats.

## Table 3

### In Vivo GH Release (ng/mL) Promoted By Growth Hormone Releasing Compounds In Pentobarbital Anesthetized Rats

### (Animals Sacrificed 10 Minutes After Final Injection)

| Column A Growth Hormone Releasing Compounds | Total Dose (µg) | Control GH ng/mL | GH Released by Compound in Column A ng/mL +SEM | p value |
|---|---|---|---|---|
| His-DTrp-Ala-Trp- | .1 | 151 ± 16 | 246 ± 39 | – |

9

| | | | | |
|---|---|---|---|---|
| DPhe-Lys-NH$_2$ | .3 | 151 $\pm$ 16 | 670 $\pm$ 75 | – |
| | 1.0 | 151 $\pm$ 16 | 1000 $\pm$ 276 | – |
| | 3.0 | 151 $\pm$ 16 | 2106 $\pm$ 216 | – |
| His-D$^\beta$Nal-Ala- | .1 | 151 $\pm$ 16 | 938 $\pm$ 255 | <.02 |
| Trp-DPhe-Lys-NH$_2$- | .3 | 151 $\pm$ 16 | 1716 $\pm$ 258 | <.02 |
| | 1.0 | 151 $\pm$ 16 | 3728 $\pm$ 691 | <.01 |
| | 3.0 | 151 $\pm$ 16 | 3238 $\pm$ 273 | <.01 |
| His-DTrp-Ala- | .1 | 138 $\pm$ 12 | 226 $\pm$ 31 | – |
| Trp-Dphe-Lys-NH$_2$ | .3 | 138 $\pm$ 12 | 613 $\pm$ 73 | – |
| | 1.0 | 138 $\pm$ 12 | 1581 $\pm$ 228 | – |
| | 3.0 | 138 $\pm$ 12 | 2875 $\pm$ 393 | – |
| Ala-His-DTrp-Ala- | .1 | 138 $\pm$ 12 | 254 $\pm$ 78 | NS – |
| Trp-DPhe-Lys-NH$_2$ | .3 | 138 $\pm$ 12 | 809 $\pm$ 59 | NS – |
| | 1.0 | 138 $\pm$ 12 | 1516 $\pm$ 215 | NS – |
| | 3.0 | 138 $\pm$ 12 | 3095 $\pm$ 473 | NS – |
| Ala-His-D$^\beta$Nal- | .1 | 138 $\pm$ 12 | 1128 $\pm$ 309 | <.02 <.02 |
| Ala-Trp-DPhe-Lys-NH$_2$ | .3 | 138 $\pm$ 12 | 2479 $\pm$ 389 | <.001 .001 |
| | 1.0 | 138 $\pm$ 12 | 3899 $\pm$ 514 | .001 .001 |
| | 3.0 | 138 $\pm$ 12 | 4202 $\pm$ 369 | <.05 NS |
| Ala-His-DTrp-Ala-Trp- | 0.1 | 111 $\pm$ 25 | 360 $\pm$ 35 | – |
| DPhe-Lys-NH$_2$ | 0.3 | 111 $\pm$ 25 | 903 $\pm$ 217 | – |
| | 1.0 | 111 $\pm$ 25 | 2957 $\pm$ 427 | – |
| | 3.0 | 111 $\pm$ 25 | 3956 $\pm$ 485 | – |
| Ala-His-D$^\beta$Nal- | 0.1 | 111 $\pm$ 25 | 970 $\pm$ 169 | <.01 |
| Ala-Trp-DPhe-Lys-NH$_2$ | 0.3 | 111 $\pm$ 25 | 2898 $\pm$ 247 | <.001 |
| | 1.0 | 111 $\pm$ 25 | 3908 $\pm$ 327 | NS |

Table 4

| In Vivo GH Release (ng/mL) Promoted By Growth Hormone Releasing Compounds In Pentobarbital Anesthetized Rats (Animals Sacrificed 10 Minutes After Final Injection) | | | |
|---|---|---|---|
| Column A Growth Hormone Releasing Compounds | Control GH ng/mL | Total Dose ($\mu$g) | GH Released by Compound in Column A ng/mL |
| AlaHisDTrpAlaTrpDPheLysNH$_2$ | 111 ± 25 | .3 | 901 ± 21 |
| AlaHisDPheAlaTrpDPheLysNH$_2$ | 181 ± 69 | .3 | 616 ± 74 |
| AlaHisDHisAlaTrpDPheLysNH$_2$ | 117 ± 19 | .3 | 268 ± 54 |
| AlaHisDProAlaTrpDPheLysNH$_2$ | 117 ± 19 | .3 | 262 ± 51 |

Table 5

| Peptide | Total ($\mu$g) Dose | Control GH ng/mL | GH Released ng/mL |
|---|---|---|---|
| His-DAsp-Ala-Trp-DPhe-Lys-NH$_2$ | 1.0 | 150 ± 20 | 154 ± 63 |
| | 3.0 | 150 ± 20 | 155 ± 34 |
| | 10.0 | 150 ± 20 | 163 ± 44 |
| | 30.0 | 150 ± 20 | 224 ± 62 |
| | 100.0 | 150 ± 20 | 178 ± 83 |
| His-DCys(SMe)-Ala-Trp-DPhe-Lys-NH$_2$ | .3 | 181 ± 69 | 178 ± 28 |
| | 1.0 | 181 ± 69 | 193 ± 28 |
| | 3.0 | 181 ± 69 | 180 ± 34 |
| His-D$^\alpha$Nal-Ala-Trp-DPhe-Lys-NH$_2$ | .1 | 151 ± 16 | 280 ± 70 |
| | .3 | 151 ± 16 | 439 ± 122 |
| | 1.0 | 151 ± 16 | 1130 ± 179 |
| | 3.0 | 151 ± 16 | 2319 ± 139 |
| His-DTrp-Ala-Trp-DPhe-Lys-NH$_2$ | .1 | 181 ± 69 | 512 ± 43 |
| | .3 | 181 ± 69 | 566 ± 114 |
| | 1.0 | 181 ± 69 | 1531 ± 303 |
| | 3.0 | 181 ± 69 | 2349 ± 267 |
| His-DTrp-Ala-Trp-DPhe-Ala-Lys-NH$_2$ | .1 | 220 ± 29 | 420 ± 105 |
| | .3 | 220 ± 29 | 900 ± 163 |
| | 1.0 | 220 ± 29 | 1965 ± 366 |
| | 3.0 | 220 ± 29 | 4553 ± 670 |
| His-D$^\beta$Nal-Ala-Trp-DPhe-Ala-Lys-NH$_2$ | .1 | 111 ± 25 | 484 ± 89 |
| | .3 | 107 ± 16 | 971 ± 241 |
| | 1.0 | 107 ± 16 | 1593 ± 359 |
| | 3.0 | 107 ± 16 | 3337 ± 583 |
| His-Ala-DTrp-Ala-Trp-DPhe-Lys-NH$_2$ | .1 | 111 ± 25 | - |
| | .3 | 151 ± 16 | 261 ± 32 |
| | 1.0 | 151 ± 16 | 830 ± 103 |
| | 3.0 | 151 ± 16 | 2588 ± 341 |

Tables 3, 4, 5 and 6 show that compounds of the invention promote the release and elevation of growth hormone levels in the blood of rats to which such compounds have been administered. The peptides of the present invention show activity whereas equivalent compounds with a substitution at the $A_2$ position (e.g. with DAsp, DCys(SMe), DHis, and DPro) do not show such activity. These tables also show that His-D$^\beta$Nal-Ala-Trp-DPhe-Lys-NH$_2$ is more active than compounds having DTrp, D$^\alpha$Nal, DAsp, and DCys (SMe) in the $A_2$ position. Similarly, Ala-His-D$^\beta$Nal-Ala-Trp-DPhe-Lys-NH$_2$ is more active than the equivalent DTrp compound.

Example 3

Administration Of A Combination Of GH-Releasing Compounds

The procedure of Example 2 was repeated, except the rats were not anesthetized nor were they pretreated with pentobarbital, and a combination of peptides were administered to the rats. The compounds administered, the dose level and results are set forth in Table 6.

Table 6

| In Vivo GH Release Prompted By Growth Hormone Releasing Compounds In Pentobarbital Anesthetized Rats (Animals Sacrificed 10 Minutes After Final Injection) | | | |
|---|---|---|---|
| Column A GH Releasing Peptide | Total Dose ($\mu$g) | Control Serum GH ng/mL ± SEM (N = 6) | GH Released Serum GH ng/ml ± SEM (N = 6) |
| Ala-His-D$\beta$Nal-Ala--Trp-DPhe-Lys-NH$_2$ | 0.1 | 337 ± 51 | 610 ± 90 |
| | 0.3 | 337 ± 51 | 1140 ± 187 |
| | 1.0 | 337 ± 51 | 2909 ± 257 |
| | 3.0 | 337 ± 51 | 3686 ± 436 |
| Ala-D$\beta$Nal-Ala-Trp--DPhe-Lys-NH$_2$ | 0.3 | 167 ± 46 | 363 ± 73 |
| | 1.0 | 167 ± 46 | 1450 ± 294 |
| | 3.0 | 167 ± 46 | 2072 ± 208 |
| | 10.0 | 167 ± 46 | 2698 ± 369 |
| Ala-His-D$\beta$Nal-Ala--Trp-DPhe-Lys-NH$_2$ | 0.3 | 160 ± 51 | 1418 ±302 |
| | 1.0 | 160 ± 51 | 2201 ±269 |
| Ala-His-D$\beta$Nal-Ala--Trp-DPhe-Lys-NH$_2$ | 0.3 | 228 ± 23 | 1746 ±318 |
| | 1.0 | 228 ± 23 | 2610 ±176 |
| Ala-His-D$\beta$Nal-Ala--Trp-DPhe-Lys-NH$_2$ | 0.1 | 160 ± 36 | 822 ± 243 |
| | 0.3 | 160 ± 36 | 1549± 292 |
| | 1.0 | 160 ± 36 | 2180± 284 |

## Table 7

### In Vivo Synergistic Effects In Unanesthetized Rats
### Of Invention Compound With Group 1 and/or Group 3 Compounds

| Compound Administered | | | Dose ($\mu$g) | GH Released (ng/mL) $\pm$ SEM |
|---|---|---|---|---|
| Control | – | – | – | 12 $\pm$ 3 |
| 175-1[a] | – | – | 1 | 58 $\pm$ 13 |
| 175-1 | – | – | 3 | 240 $\pm$ 32 |
| C[b] | – | – | 10 | 204 $\pm$ 50 |
| GHRH[c] | – | – | 3 | 131 $\pm$ 50 |
| TP[d] | – | – | 10 | 79 $\pm$ 29 |
| 175-1 | GHRH | – | 1 + 3 | 2014 $\pm$ 224 |
| 175-1 | TP | – | 1 + 10 | 987 $\pm$ 204 |
| 175-1 | GHRH | TP | 1 + 3 + 10 | 4150 $\pm$ 555 |
| 175-1 | GHRH | – | 3 + 3 | 1994 $\pm$ 249 |
| 175-1 | TP | – | 3 + 10 | 2149 $\pm$ 451 |
| 175-1 | GHRH | TP | 3 + 3 + 10 | 2922 $\pm$ 384 |
| C | GHRH | – | 10 + 3 | 2525 $\pm$ 453 |
| C | TP | – | 10 + 10 | 1597 $\pm$ 387 |
| C | GHRH | TP | 10 + 3 + 10 | 4344 $\pm$ 374 |

* Groups 1 and 3 compounds are described in detail in U.S. Patent No. 4,880,778.

a – 175-1 = His-D$^\beta$Nal-Ala-Trp-DPhe-Lys-NH$_2$ (Compound within invention)

b – C = His-DTrp-Ala-Trp-DPhe-Lys-NH$_2$ (Comparison compound)

c – GHRH = Tyr-Ala-Asp-Ala-Ile-Phe-
Thr-Asn-Ser-Tyr-Arg-Lys-
Val-Leu-Gly-Gln-Leu-Ser-
Ala-Arg-Lys-Leu-Leu-Gln-
Asp-Ile-Nle-Ser-Arg-NH$_2$
(Group 1 Compound)

d – TP = Tyr-DArg-Phe-Gly-NH$_2$ (Group 3 Compound)

Table 7 shows that the invention compound displays a synergistic response when administered with exemplary Group 1 and/or Group 3 compounds. The results in Table 7 further show that the invention compound has a greater synergistic response that that obtained with a comparison compound (C), which has previously been shown to have a synergistic response.

EXAMPLE 4

Condensation Reaction of Peptide Fragments To Form Peptide

General Procedures

Melting points can be determined using a Thomas Hoover capillary melting point apparatus. Infrared (IR) spectra can be recorded on a Perkin-Elmer Model 137 or a Nicolet Model 5DX spectrophotometer and reported in wave numbers (cm$^{-1}$) . Mass spectra (MS) can be obtained using a VG Analytical Ltd. Model ZAB-IF Mass Spectrometer in EI (electron impact), FD (field desorption) or FAB (fast atom bombardment) modes. GCMS can be obtained using a Finnigan 4023 GCMS equipped with a 30 m D85 capillary column (J & W Scientific) using helium carrier gas. Optical rotations can be measured using an Autopol III polarimeter manufactured by Rudolph Research.

$^1$H NMR spectra can be obtained on a JEOL GX-400 NMR instrument operating at 400 MHz or a JEOL GX-270 NMR instrument operating at 270 MHz. These instruments are capable of a routine digital resolution of less than 0.7 Hz. Chemical shifts are expressed in parts per million relative to internal 3-(trimethylsilyl)-tetradeutero sodium propionate (TSP).

High performance liquid chromatography (HPLC) can be accomplished using a Hitachi system consisting of a L-5000 gradient controller and a 655A pump attached to a Vydac 201TP1010 or 218TP1010 semipreparative column. Combinations of water containing 0.2% trifluoroacetic acid and methanol can be used as the eluting solvent. Typically, compounds of interest will be eluted at a flow rate of six mL per minute with a gradient increasing the organic component at a rate of approximately 1-2% per minute. Compounds are then detected at appropriate wavelengths using an LKB 2140 diode array U.V. detector. Integrations can then be accomplished using Nelson Analytical software (Version 3.6).

Reactions will be carried out under an inert atmosphere of nitrogen or argon unless otherwise specified. Anhydrous tetrahydrofuran (THF, U.V. grade) and dimethylformamide (DMF) can be purchased from Burdick and Jackson and used directly from the bottle.

A. Preparation of Tripeptide Fragment-K-His-D$\underline{\beta}$Nal-Ala-OMe

N$\underline{\alpha}$-benzyloxycarbonyl-histidyl-D-beta-napthyl alanine methyl ester, 25.

A solution of EtOAc (400 mL) and D-beta-napthylalanine methyl ester hydrochloride ($\underline{22}$, 0.62 mol) are washed with saturated sodium carbonate (400 mL) and 0.8 $\underline{N}$ aqueous sodium hydroxide (ca. 500 mL). The resulting aqueous phase is removed (pH 8.5) and the organic phase is sequentially washed with half-saturated aqueous $Na_2CO_3$ (150 mL) and then with water (50 mL) . The free base form of $\underline{22}$ is isolated upon concentration of the ethyl acetate layer in vacuo.

Dicyclohexylcarbodiimide (DCC, ca. 95 g, 0.46 mol) is added to a -5°C (ice-ethanol bath) solution of N$^\alpha$-benzyloxycarbonyl-histidine ($\underline{19}$, 143.5 g, 0.50 mol), N-hydroxysuccinimide (HONSu, $\underline{23}$, 0.62 mol) and the freshly prepared-free base form of $\underline{22}$ (ca. 0.52 mol) in DMF (Ca. 3L). The resulting reaction solution is allowed to stir for 24 hours while warming to room temperature. HPLC analysis should be used to see if the reaction is complete. If it is not, the reaction solution is then cooled to ca. -5°C and an additional portion of dicyclohexylcarbodiimide (ca. 0.17 mol) is added to the reaction. The reaction mixture is then allowed to stir for an additional 24 hours while warming to room temperature. The mixture is then filtered to remove dicyclohexylurea (DCU). Water (1 L) is added to the filtrate and the resulting solution is concentrated in vacuo. The resulting residue is taken up in aqueous 1$\underline{N}$ HCl (ca. 1 L until the pH of the aqueous phase reaches a pH of 1). The aqueous phase is then extracted with two portions of ethyl acetate (1 L each). The ethyl acetate layers are discarded. The pH of the aqueous phase is then adjusted by addition of cold 2$\underline{N}$ sodium hydroxide (500 mL) and sodium hydroxide pellets. During this neutralization, the solution is kept cold by addition of cold ethyl acetate (1 L). When the pH of the aqueous phase reaches approximately 7, copious precipitation of a white solid or oil usually results. This precipitate is collected by vacuum filtration or decantation and washed sequentially with half saturated sodium carbonate (2 X 1500 mL), water (6 X 1500 mL) and ethyl acetate (3 X 1500 mL). The resulting material is dried under high vacuum to constant weight. This material can be hydrolyzed directly without further purification.

The DPhe peptide can be prepared using D-phenylalanine methyl ester hydrochloride as compound $\underline{22}$ instead of D-beta-napthylalalanine methyl ester hydrochloride.

N$^\alpha$-Benzyloxycarbonyl-histidyl-$\beta$-napthyl-D-alanine , 26.

Aqueous sodium hydroxide (192 mL, 0.08 g/mL solution, 0.38 mol) is added to a solution of dipeptide 25 (ca. 0.38 mol), water (360 mL) and MeOH (ca. 6 L). The solution is stirred at room temperature until hydrolysis is complete (ca. 24 hours). The disappearance of the starting peptide is established by HPLC analysis. The solution is concentrated in vacuo to a residue which is dissolved in water (ca. 1 L). The aqueous layer (pH ca. 10) is then extracted with EtOAc (2 X 500 mL) in a separatory funnel. The ethyl acetate layers are discarded. The resulting aqueous phase is adjusted to a pH of approximately 5 with concentrated HCl at which point precipitation of a white solid or oil usually results. The product is collected and is dried in vacuo.

N$^\alpha$-Benzyloxycarbonyl-histidyl-D-beta-napthyl alanyl-alanine methyl ester, 20.

The dipeptide N$^\alpha$-benzyloxycarbonyl-histidyl-D-beta-napthyl alanine (26, 0.253 mol) is added to a solution of the HONSu (23, 0.505 mol) in DMF (800 mL) under an atmosphere of argon. To this solution, a mixture of alanine methyl ester hydrochloride (15, 0.303 mol), N-methylmorpholine (16, 0.303 mol) and DMF (200 mL) is added. The resulting solution is cooled to 10°C, at which time dicyclohexylcarbodiimide (24, 0.265 mol) in methylene chloride (273 mL) is added. The reaction is monitored by HPLC while the reaction temperature is maintained at 10°C until the reaction is complete. If after several days (ca. 4), the reaction has not progressed to completion, an additional charge of 24 (0.080 mol) is added and the reaction mixture is allowed to stir for an additional day at 10°C. The reaction is again monitored by HPLC analysis until complete (typically ca. 5 days). The solids which form during the reaction are collected by vacuum filtration. The filtrate is then concentrated to a residue in vacuo. The resulting residue is taken up in ethyl acetate and extracted with half-saturated aqueous Na$_2$CO$_3$ (2 X 500 mL). The ethyl acetate phase is dried over MgSO$_4$. The resulting solution is clarified and is concentrated to a residue in vacuo.

B. Preparation of Tetrapeptide Fragment Ala-His-D$^\beta$Nal-Ala-OH Histidyl-D-beta-Napthyl-Alanyl-Alanine Methyl Ester, 30.

Five percent palladium on carbon (3 g) is carefully added to a solution of N$^\alpha$-benzyloxycarbonyl-histidyl-D-beta-napthyl alanyl-alanine methyl ester 20 (71 mmol) in methanol (500 mL) under an argon atmosphere. Argon is bubbled through the reaction mixture for 15 minutes and acetic acid (15 mL, 0.26 mol) is then added. Hydrogen is bubbled (subsurface) through the resulting mixture for 15 minutes and then the reaction is allowed to stir at room temperature under a hydrogen ballast (1 atm). HPLC analysis is then used to monitor how much, if any, starting material remains. If some remains, one carefully bubbles argon through the reaction mixture (subsurface for 30 minutes), and an additional portion of 5% palladium on carbon (2.5 g) is added. Hydrogen is then reintroduced. Argon is again bubbled through the reaction mixture and the resulting solution is clarified by filtration through a pad of diatomaceous earth. The resulting solution is concentrated in vacuo to provide the product. A portion of this product is dissolved or slurried in water (500 mL). Ethyl acetate and saturated aqueous sodium carbonate are added to the resulting material. Isolation of the ethyl acetate layer or the solids which result, provides a material (30) which is free of acetate.

Boc-Alanine N-hydroxysuccinimide ester, 31.

Dicyclohexylcarbodiimide (43 mmol) is added to a room temperature solution of Boc-alanine (43 mmol) and N-hydroxysuccinimide (48 mmol) in methylene chloride (250 mL). The resulting solution is allowed to stir overnight. The reaction mixture is then filtered to remove dicyclohexylurea and the clarified filtrate is concentrated in vacuo to provide the product, which is stored at -20°C under an argon atmosphere prior to use.

Boc-Ala-His-D-$^\beta$Nal-Ala-OMe, 32.

The above alanine ester (23.9 mmol) is added to a solution of His-D-$^\beta$Nal-Ala-OMe (30) (20.1 mmol) in anhydrous dimethylformamide (DMF, 200 mL). The resulting homogeneous solution is allowed to stir over the weekend at room temperature and monitored. When HPLC analysis indicates that virtually no tripeptide remains in the reaction mixture (e.g., about 3 days), water (50 mL) is added to the reaction and the resulting mixture is allowed to stir for an additional day. This solution is then concentrated in vacuo. The resulting residue is dissolved in ethyl acetate and is then extracted with half-saturated aqueous sodium carbonate (2 x 300 mL). The organic phase is dried with MgSO₄ and Na₂SO₄ and is concentrated in vacuo to provide the tetrapeptide. This material is used without further purification in the preparation of Boc-Ala-His-D-$^\beta$Nal-Ala-OH.

Boc-Ala-His-D-$^\beta$Nal-Ala-OH, 33.

A 2N aqueous sodium hydroxide solution (7.5 mL, 15 mmol) is added to a methanol (500 mL) and water solution (200 mL) containing Boc-Ala-His-D-$^\beta$Nal-Ala-OMe (13.7 mmol). After the reaction is allowed to stir overnight at room temperature, HPLC analysis indicates the amount of the starting material remaining. When it is essentially complete (ca. overnight), the resulting solution is concentrated in vacuo to a volume of approximately 200 mL. Water (100 mL) is added and the pH is adjusted to approximately 12 by addition of 2N sodium hydroxide (1 mL). The resulting solution is extracted with ethyl acetate (2 X 500 mL). The ethyl acetate layers are discarded. The pH of the aqueous phase is then adjusted to approximately 5 by addition of aqueous HCl which usually results in the precipitation of the product. It is important to minimize the volume of the aqueous phase to promote this precipitation. The aqueous phase is decanted away from the product and the product is then rinsed with water (2 X 50 mL). The isolated product is dried to constant weight in vacuo.

C. Condensation Reaction Of Peptide Fragments To Produce Heptapeptide Boc-Ala-His-D$^\beta$Nal-Ala-Trp-D-Phe-Lys(Boc)-NH₂, 34.

The two peptides Boc-Ala-His-D-$\beta$Nal-Ala-OH (33, 2.6 mmol) and Trp-D-Phe-Lys(Boc)-NH₂ (13, 2.8 mmol) are dissolved in anhydrous DMF and the resulting solution is concentrated in vacuo. This preliminary concentration is carried out in an attempt to remove any traces of methanol which might be present. The resultant peptide mixture is redissolved in DMF and N-hydroxysuccinimide (5.1 mmol) is then added. The resulting solution is then cooled to a solution temperature of -2°C and dicyclohexylcarbodiimide (3.4 mmol) is then added as a solution in methylene chloride (3.5 mL). The resulting reaction mixture is allowed to stir at -2°C solution temperature for a period of three days. HPLC analysis is used to determine if the reaction is essentially completed. After this period of time, if it is not, additional dicyclohexylcarbodiimide can then be added and the resultant reaction mixture allowed to stir for an additional day at -2°C. If, on the following day (for a total of four days) HPLC analysis again indicates incomplete reaction, cooling of the reaction mixture should be terminated. The solution temperature of the reaction can be allowed to slowly rise to room temperature (25°C) over a period of hours (ca. 8). The resultant reaction mixture is allowed to stir overnight at room temperature. The procedure is repeated until the reaction is complete. Then, water (50 mL) is added and the resulting mixture is allowed to stir for an additional day. The reaction solution is then filtered to remove dicyclohexylurea and the resulting filtrate is concentrated in vacuo to a viscous oil. Ethyl acetate and half-saturated aqueous sodium carbonate (200 mL) are added to the resulting residue. The two-phase mixture is vigorously swirled on a rotary evaporator for approximately one hour. Any solids formed are collected to provide the product by filtration on a scintered glass funnel. The organic phase is washed with water and then dried to constant weight in vacuo to provide the product.

Ala-His-D-$^\beta$Nal-Ala-Trp-D-Phe-Lys-NH₂, 35.

The heptapeptide Boc-Ala-His-D-$^\beta$Nal-Ala-Trp-D-Phe-Lys-(Boc)-NH₂ (34, 1.02 mmol) is added to a room temperature solution of trifluoroacetic acid (30 mL), dimethylsulfide (14 mL), 1,2 ethanedithiol (7 mL) and anisole (2.2 mL) in methylene chloride (15 mL). The homogeneous reaction mixture is allowed to stir for 15 minutes. After this period of time, anhydrous ether (450 mL) is added to cause precipitation of the crude biologically active peptide product 35. This product is isolated by filtration on a scintered glass funnel or by decantation. The resultant product is dissolved in water and lyophilized. The lyophilized product can be further purified by medium pressure chromatography on a 26 X 460 mm glass column containing Lichroprep™ RP-18 column packing material (C-18, 25-40 nm, irregular mesh). After injection of the peptide as a solution in water, the column is eluted at a flow rate of 9 mL per minute with a shallow gradient of 0 to 25% methanol for 5-20 hours, and then by a gradient of 25 to 55% methanol over ca. 48 hours. The methanol concentration of the gradient is then increased at a rate of 2% per hour. During the elution, the remainder of the solvent composition is made up of water containing 0.2% trifluoroacetic acid. The product (35) is identified by HPLC and is isolated by concentration of the appropriate elution volumes.

16

**Claims**

1. A peptide of the formula

A$_1$-A$_2$-A$_3$-Trp-A$_5$-A$_5$-Z,

wherein A$_1$ is His, 3(NMe)His, His-Ala, Ala, Tyr, A$_0$-His or A$_0$-3(NMe)His, where A$_0$ is any naturally occurring L-amino acid, Met(O), DOPA, Abu or peptides of the formula L-A$_0$, wherein L is H, DOPA, Lys, Phe, Tyr, Cys, Tyr-DAla-Phe-Gly, Tyr-DAla-Gly-Phe, Tyr-Ala-Gly-Thr or Tyr-DAla-Phe-Sar;

A$_2$ is D$^\beta$Nal;

A$_3$ is Ala, Gly or Ser;

A$_5$ is DPhe, D/L$^\beta$(Me)Phe or (NMe)DPhe;

A$_5$ is B-G or G, wherein B is any naturally occurring L-amino-acid, dipeptides of any naturally occurring amino acids or H$_2$N(CH$_2$)$_n$CO$_2$H, wherein n = 2-12, and G is Arg, iLys, Lys or Orn;

Z is the C terminal end group of the polypeptide or the C terminal amino acid(s) plus end group, and Z is -CONR$^1$R$^2$, -COOR$^1$, -CH$_2$OR$^1$, -Gly-Z' , -Met-Z' -Lys-Z' , -Cys-Z' , -Gly-Tyr-Z' , or -Ala-Tyr-Z', wherein Z' is -CONR$^1$R$^2$, -COOR$^1$ or -CH$_2$OR$^1$, wherein R$^1$ is H, an alkyl group having from 1 to about 6 carbon atoms, a cycloalkyl group having from 3 to about 8 carbon atoms, an alkenyl group having from 2 to about 8 carbon atoms, or an aryl group having from 6 to about 12 carbon atoms; R$^2$ is defined as R$^1$ and may be the same or different; wherein amino acid residue abbreviations are in accordance with those recommended by the IUPAC-IUB Commission on Biochemical Nomenclature or those conventionally used in the art, unless otherwise specified the amino acid is in the L form, where the three letter amino acid abbreviation is preceded by a D, the amino acid is in the D form, where it is preceded by a D/L, the amino acid is a mixture of the D and L configurations;

DOPA = 3, 4-dihydroxyphenylalanine, Met(O) = methionine sulfoxide, Abu = $\alpha$-aminobutyric acid, iLys = N$^\epsilon$-isopropyl-L-lysine, 4-Abu = 4-aminobutyric acid, Orn = L-ornithine, D$^\alpha$Nal = $\alpha$-naphthyl-D-alanine. D$^\beta$Nal = $\beta$-naphthyl-D-alanine, Sar = sarcosine, (Me) = methyl, (NMe) = N-methyl;

and the organic or inorganic pharmaceutically acceptable salts thereof.

2. The peptide according to claim 1, wherein A$_1$ is His, Ala, His-Ala or A$_0$-His.

3. The peptide according to claim 1, wherein A$_3$ is Ala.

4. The peptide according to claim 2, wherein A$_0$ is Ala, Lys, or Glu; A$_3$ is Ala; A$_5$ is Lys, iLys, Ala-Lys, Ala-Ala-Lys or H$_2$N(CH$_2$)$_n$,CO-Lys, where n' is 4-8.

5. The peptide according to claim 1 having the formula

His-D$^\beta$Nal-Ala-Trp-DPhe-A$_5$-Z.

6. The peptide according to claim 5 having the formula

His-D$^\beta$Nal-Ala-Trp-DPhe-Lys-NH$_2$.

7. The peptide according to claim 1 having the formula

A$_0$-His-D$^\beta$Nal-Ala-Trp-DPhe-A$_5$-Z .

8. The peptide according to claim 7, wherein A$_0$ is Ala.

9. The peptide according to anyone of claims 5, 7, 12 or 14 wherein Z in -CONH$_2$.

10. The peptide according to claim 1, having the formula

Ala-His-D$^\beta$Nal-Ala-Trp-DPhe-Lys-NH$_2$,
Ala-His-D$^\beta$Nal-Ala-Trp-DPhe-Ala-Lys-NH$_2$,
Ala-His-D$^\beta$Nal-Ala-Trp-DPhe-Ala-Ala-Lys-NH$_2$,
Lys-His-D$^\beta$Nal-Ala-Trp-DPhe-A$_5$-NH$_2$, or

17

Glu-His-D$^\beta$Nal-Ala-Trp-DPhe-A$_6$-NH$_2$.

11. The peptide according to claims 2, 5, 7 or 14, wherein A$_6$ is G.

12. The peptide according to claim 1 having the formula

His-Ala-D$^\beta$Nal-Ala-Trp-DPhe-A$_6$-Z.

13. The peptide according to claim 12 having the formula

His-Ala-D$^\beta$Nal-Ala-Trp-DPhe-Lys-NH$_2$.

14. The peptide according to claim 1 having the formula

Ala-D$^\beta$Nal-Ala-Trp-DPhe-A$_6$-Z.

15. The peptide according to claim 14 having the formula

Ala-D$^\beta$Nal-Ala-Trp-DPhe-Lys-NH$_2$.

16. A non-therapeutic method of promoting the release of growth hormone and increasing its levels in a non-human animal comprising administering to the animal an effective amount of at least one of the peptides of claims 1,4,7,10 or 12.

17. The method of claim 16, wherein the non-human animal is a mammal.

18. A pharmaceutical composition for promoting the release of growth hormone and increasing its levels in animals comprising at least one of the peptides of claim 1, 4, 7, 10 or 12 and a pharmaceutically acceptable carrier or diluent.

19. The peptide according to claim 2, wherein A$_0$ is any naturally occurring amino acid.

20. A synergistic combination of the peptide of claim 1 and a second compound, wherein said second compound is a compound which acts as an agonist at the growth hormone releasing hormone receptor or inhibits the release of somatostatin, adapted for promoting the release and elevation of blood growth hormone levels.

21. The pharmaceutical compositions of claim 18, which further comprises a second compound which acts as an agonist at the growth hormone releasing hormone receptor or inhibits the release of somatostatin.

22. The use of a compound of the formula:

A$_1$-A$_2$-A$_3$-Trp-A$_6$-A$_6$-Z,

wherein A$_1$ is His, 3 (NMe) His, His-Ala, Ala, Tyr, A$_0$-His or A$_0$-3(NMe)His, where A$_0$ is any naturally occurring L-amino acid, Met(O), DOPA, Abu or peptides of the formula L-A$_0$, wherein L is H, DOPA, Lys, Phe, Tyr, Cys, Tyr-DAla-Phe-Gly, Tyr-DAla-Gly-Phe, Tyr-Ala-Gly-Thr or Tyr-DAla-Phe-Sar;
A$_2$ is D$^\beta$Nal;
A$_3$ is Ala, Gly or Ser;
A$_6$ is DPhe, D/L$^\beta$(Me)Phe or (NMe)DPhe;
A$_6$ is B-G or G, in the manufacture of a medicament for promoting the release of blood growth hormone and elevating its levels.

23. A process for the preparation of a compound of the formula

A$_1$-A$_2$-A$_3$-Trp-A$_6$-A$_6$-Z,

wherein A$_1$ is His, 3(NMe)His, His-Ala, Ala, Tyr, A$_0$-His or A$_0$-3(NMe)His, where A$_0$ is any naturally

occurring L-amino acid, Met(O), DOPA, Abu or peptides of the formula L-$A_0$, wherein L is H, DOPA, Lys, Phe, Tyr, Cys, Tyr-DAla-Phe-Gly, Tyr-DAla-Gly-Phe, Tyr-Ala-Gly-Thr or Tyr-DAla-Phe-Sar;

$A_2$ is $D^\beta$Nal;

$A_3$ is Ala, Gly or Ser;

$A_5$ is DPhe, D/L$^\beta$(Me)Phe or (NMe)DPhe;

$A_5$ is B-G or G, which comprises coupling amino acids or amino acid derivatives to form the compound.

**24.** A process for the preparation of the peptide of claim 1 which comprises a condensation reaction of peptide fragments to form the peptide.


**Patentansprüche**


**1.** Peptid der Formel

$A_1$-$A_2$-$A_3$-Trp-$A_5$-$A_5$-Z,

wobei $A_1$ His, 3 (NMe) His, His-Ala, Ala, Tyr, $A_0$-His oder $A_0$-3 (NMe) His ist, wobei $A_0$ jede beliebige natürlich vorkommende L-Aminosäure, Met(O), DOPA, Abu oder Peptide der Formel L-$A_0$ ist, wobei L H, DOPA, Lys, Phe, Tyr, Cys, Tyr-DAla-Phe-Gly, Tyr-DAla-Gly-Phe, Tyr-Ala-Gly-Thr oder Tyr-DAla-Phe-Sar ist;

$A_2$ $D^\beta$Nal ist;

$A_3$ Ala, Gly oder Ser ist;

$A_5$ DPhe, D/L$^\beta$(Me)Phe oder (NMe)DPhe ist;

$A_5$ B-G oder G ist, wobei B jede beliebige natürlich vorkommende L-Aminosäure, Dipeptide aller beliebigen natürlich vorkommenden Aminosäuren oder $H_2N$ $(CH_2)_nCO_2H$ ist, wobei n = 2 - 12, und G Arg, iLys, Lys oder Orn ist;

Z die C-terminale Endgruppe des Polypeptids oder die C-terminale Aminosäure(n) plus Endgruppe ist, und Z -$CONR^1R^2$, -$COOR^1$, -$CH_2OR^1$, -Gly-Z', -Met-Z', -Lys-Z', -Cys-Z', -Gly-Tyr-Z' oder -Ala-Tyr-Z' ist, wobei Z' -$CONR^1R^2$, -$COOR^1$ oder -$CH_2OR^1$ ist, wobei $R^1$ H, eine Alkylgruppe mit von 1 bis etwa 6 Kohlenstoffatomen, eine Cycloalkylgruppe mit von 3 bis etwa 8 Kohlenstoffatomen, eine Alkenylgruppe mit von 2 bis etwa 8 Kohlenstoffatomen oder eine Arylgruppe mit von 6 bis etwa 12 Kohlenstoffatomen ist; $R^2$ wie $R^1$ definiert ist und gleich oder verschieden sein kann; wobei Aminosäurerestabkürzungen mit den von der IUPAC-IUB-Kommission für biochemische Nomenklatur oder den gewöhnlich in diesem Gebiet verwendeten übereinstimmen, sofern nicht anders angegeben, die Aminosäure in der L-Form vorliegt, wenn der Drei-Buchstaben-Aminosäureabkürzung ein D vorangestellt ist, die Aminosäure in der D-Form vorliegt, wenn ein D/L vorangestellt ist, die Aminosäure eine Mischung von D- und L-Konfigurationen ist;

DOPA = 3, 4-Dihydroxyphenylalanin, Met(O) = Methioninsulfoxid, Abu = $\alpha$-Aminobuttersäure, iLys = $N^\epsilon$-Isopropyl-L-Lysin, 4-Abu = 4-Aminobuttersäure, Orn = L-Ornithin, $D^\alpha$Nal = $\alpha$-Naphthyl-D-Alanin, $D^\beta$Nal = $\beta$-Naphthyl-D-Alanin, Sar = Sarkosin, (Me) = Methyl, (NMe) = N-Methyl; und die organischen oder anorganischen pharmazeutisch akzeptablen Salze derselben.


**2.** Peptid gemäß Anspruch 1, wobei $A_1$ His, Ala, His-Ala oder $A_0$-His ist.


**3.** Peptid gemäß Anspruch 1, wobei $A_3$ Ala ist.


**4.** Peptid gemäß Anspruch 2, wobei $A_0$ Ala, Lys oder Glu ist; $A_3$ Ala ist; $A_5$ Lys, iLys, Ala-Lys, Ala-Ala-Lys oder $H_2N$ $(CH_2)_{n'}CO$-Lys ist, wobei n' 4-8 ist.


**5.** Peptid gemäß Anspruch 1 mit der Formel

His-$D^\beta$Nal-Ala-Trp-DPhe-$A_5$-Z.


**6.** Peptid gemäß Anspruch 5 mit der Formel

His-$D^\beta$Nal-Ala-Trp-DPhe-Lys-$NH_2$.

EP 0 540 676 B1

**7.** Peptid gemäß Anspruch 1 mit der Formel

$A_0$-His-D$^\beta$Nal-Ala-Trp-DPhe-A$_6$-Z.

**8.** Peptid gemäß Anspruch 7, wobei $A_0$ Ala ist.

**9.** Peptid gemäß einem der Ansprüche 5, 7, 12 oder 14, wobei Z -CONH$_2$ ist.

**10.** Peptid gemäß Anspruch 1 mit der Formel

Ala-His-D$^\beta$Nal-Ala-Trp-DPhe-Lys-NH$_2$,
Ala-His-D$^\beta$Nal-Ala-Trp-DPhe-Ala-Lys-NH$_2$,
Ala-His-D$^\beta$Nal-Ala-Trp-DPhe-Ala-Ala-Lys-NH$_2$,
Lys-His-D$^\beta$Nal-Ala-Trp-DPhe-A$_6$-NH$_2$ oder
Glu-His-D$^\beta$Nal-Ala-Trp-DPhe-A$_6$-NH$_2$.

**11.** Peptid gemäß Anspruch 2, 5, 7 oder 14, wobei $A_6$ G ist.

**12.** Peptid gemäß Anspruch 1 mit der Formel

His-Ala-D$^\beta$Nal-Ala-Trp-DPhe-A$_6$-Z.

**13.** Peptid gemäß Anspruch 12 mit der Formel

His-Ala-D$^\beta$Nal-Ala-Trp-DPhe-Lys-NH$_2$.

**14.** Peptid gemäß Anspruch 1 mit der Formel

Ala-D$^\beta$Nal-Ala-Trp-DPhe-A$_6$-Z.

**15.** Peptid gemäß Anspruch 14 mit der Formel

Ala-D$^\beta$Nal-Ala-Trp-DPhe-Lys-NH$_2$.

**16.** Nicht therapeutisches verfahren zur Förderung der Freisetzung von Wachstumshormon und Steigerung seiner Spiegel in einem nicht-humanen Tier, umfassend die Verabreichung einer wirksamen Menge wenigstens eines der Peptide nach Anspruch 1, 4, 7, 10 oder 12 an das Tier.

**17.** Verfahren nach Anspruch 16, wobei das nicht-humane Tier ein Säugetier ist.

**18.** Pharmazeutische Zusammensetzung zur Förderung der Freisetzung von Wachstumshormon und Steigerung seiner Spiegel in Tieren, umfassend wenigstens eines der Peptide nach Anspruch 1, 4, 7, 10 oder 12 und einen pharmazeutisch akzeptablen Träger- oder Verdünnungsstoff.

**19.** Peptid gemäß Anspruch 2, wobei $A_0$ jede beliebige natürlich vorkommende Aminosäure ist.

**20.** Synergistische Kombination des Peptids nach Anspruch 1 und einer zweiten Verbindung, wobei die zweite Verbindung eine Verbindung ist, die als ein Agonist an dem Wachstumshormonfreisetzungshormonrezeptor wirkt oder die Freisetzung von Somatostatin hemmt und zur Förderung der Freisetzung und Erhöhung der Blut-Wachstumshormonspiegel eingerichtet ist.

**21.** Pharmazeutische Zusammensetzung nach Anspruch 18, die ferner eine zweite Verbindung umfaßt, die als ein Agonist am Wachstumshormonfreisetzungshormonrezeptor wirkt oder die Freisetzung von Somatostatin hemmt.

**22.** Verwendung einer Verbindung der Formel:

$A_1$-$A_2$-$A_3$-Trp-A$_6$-A$_6$-Z,

20

wobei $A_1$ His, 3 (NMe) His, His-Ala, Ala, Tyr, $A_0$-His oder $A_0$-3(NMe)His ist, wobei $A_0$ jede beliebige natürlich vorkommende L-Aminosäure, Met(O), DOPA, Abu oder Peptide der Formel L-$A_0$ ist, wobei L H, DOPA, Lys, Phe, Tyr, Cys, Tyr-DAla-Phe-Gly, Tyr-DAla-Gly-Phe, Tyr-Ala-Gly-Thr oder Tyr-DAla-Phe-Sar ist;

$A2$ $D^\beta$Nal ist;

$A_3$ Ala, Gly oder Ser ist;

$A_5$ DPhe, D/L$^\beta$(Me)Phe oder (NMe) DPhe ist;

$A_5$ B-G oder G ist, bei der Herstellung eines Medikamentes zur Förderung der Freisetzung von Blutwachstumshormon und zur Erhöhung seiner Spiegel.

23. Verfahren zur Herstellung einer Verbindung der Formel

$A_1$-$A_2$-$A_3$-Trp-$A_5$-$A_5$-Z,

wobei $A_1$ His, 3(NMe)His, His-Ala, Ala, Tyr, $A_0$-His oder $A_0$-3(NMe)His ist, wobei $A_0$ jede beliebige natürlich vorkommende L-Aminosäure, Met(O), DOPA, Abu oder Peptide der Formel L-$A_0$ ist, wobei L H, DOPA, Lys, Phe, Tyr, Cys, Tyr-DAla-Phe-Gly, Tyr-DAla-Gly-Phe, Tyr-Ala-Gly-Thr oder Tyr-DAla-Phe-Sar ist;

$A_2$ $D^\beta$Nal ist;

$A_3$ Ala, Gly oder Ser ist;

$A_5$ DPhe, D/L$^\beta$(Me)Phe oder (NMe)DPhe ist;

$A_5$ B-G oder G ist, das das Kopplung von Aminosäuren oder Aminosäurederivaten zur Bildung der Verbindung umfaßt.

24. Verfahren zur Herstellung des Peptids nach Anspruch 1, das eine Kondensationsreaktion von Peptidfragmenten zur Bildung des Peptids umfaßt.

**Revendications**

1. Peptide de formule

$A_1$-$A_2$-$A_3$-Trp-$A_5$-$A_5$-Z

dans laquelle $A_1$ représente un groupe His, 3(NMe)His, His-Ala, Ala, Tyr, $A_0$-His ou $A_0$-3(NMe)His, dans lequel $A_0$ représente n'importe quel L-amino-acide naturel, Met(O), DOPA, Abu ou des peptides de formules L-$A_0$, dans laquelle L représente H, DOPA, Lys, Phe, Tyr, Cys, Tyr-DAla-Phe-Gly, Tyr-DAla-Gly-Phe, Tyr-Ala-Gly-Thr ou Tyr-DAla-Phe-Sar ;

$A_2$ représente $D^\beta$Nal ;

$A_3$ représente Ala, Gly ou Ser ;

$A_5$ représente DPhe, D/L$^\beta$(Me)Phe ou (NMe)DPhe ;

$A_5$ représente B-G ou G, dans lequel B représente n'importe quel L-amino-acide naturel, des dipeptides de n'importe quels amino-acides naturels ou un groupe de formule $H_2N(CH_2)_nCO_2H$, dans laquelle n a une valeur de 2 à 12, et G représente Arg, iLys, Lys ou Orn ;

Z représente le groupe C-terminal à l'extrémité du polypeptide ou le ou les amino-acides C-terminaux plus le groupe terminal, et Z représente un groupe -CONR$^1$R$^2$, - COOR$^1$, -CH$_2$OR$^1$, -Gly-Z', -Met-Z', -Lys-Z', -Cys-Z', - Gly-Tyr-Z' ou -Ala-Tyr-Z', dans lequel Z' représente un groupe -CONR$^1$R$^2$, -COOR$^1$ ou -CH$_2$OR$^1$, dans lequel R$^1$ représente H, un groupe alkyle ayant 1 à environ 6 atomes de carbone, un groupe cycloalkyle ayant 3 à environ 8 atomes de carbone, un groupe alcényle ayant 2 à environ 8 atomes de carbone ou un groupe aryle ayant 6 à environ 12 atomes de carbone ; R$^2$ répond à la définition de R$^1$ et peut être identique à, ou différent de, ce dernier ; les abréviations des résidus d'amino-acides étant conformes à celles recommandées par la IUPAC-IUB Commission on Biochemical Nomenclature ou à celles utilisées classiquement dans la pratique ; sauf spécification contraire, l'amino-acide est sous forme L ; lorsque l'abréviation d'amino-acide en trois lettres est précédée par un D, l'amino-acide est sous forme D ; lorsqu'elle est précédée par D/L, l'amino-acide consiste en un mélange des configurations D et L ;

DOPA = 3,4-dihydroxyphénylalanine, Met(O) = méthioninesulfoxyde, Abu = acide $\alpha$-aminobutyrique, iLys = N$^\epsilon$-isopropyl-L-lysine, 4-Abu = acide 4-aminobutyrique, Orn = L-ornithine, D$^\alpha$Nal = $\alpha$-

naphtyl-D-alanine, $D^\beta$Nal = $\beta$-naphtyl-D-alanine, Sar = sarcosine, (Me) = méthyle, (NMe) = N-méthyle ;

et ses sels organiques ou inorganiques pharmaceutiquement acceptables.

2. Peptide suivant la revendication 1, dans lequel $A_1$ représente His, Ala, His-Ala ou $A_0$-His.

3. Peptide suivant la revendication 1, dans lequel $A_3$ représente Ala.

4. Peptide suivant la revendication 2, dans lequel $A_0$ représente Ala, Lys ou Glu ; $A_3$ représente Ala ; $A_6$ représente Lys, iLys, Ala-Lys, Ala-Ala-Lys ou $H_2N(CH_2)_{n'}CO$-Lys, dans lequel n' a une valeur de 4 à 8.

5. Peptide suivant la revendication 1, répondant à la formule His-$D^\beta$Nal-Ala-Trp-DPhe-$A_6$-Z.

6. Peptide suivant la revendication 5, répondant à la formule His-$D^\beta$Nal-Ala-Trp-Dphe-Lys-NH$_2$

7. Peptide suivant la revendication 1, répondant à la formule $A_0$-His-$D^\beta$nal-Ala-Trp-DPhe-$A_6$-Z.

8. Peptide suivant la revendication 7, dans lequel $A_0$ représente Ala.

9. Peptide suivant l'une quelconque des revendications 5, 7, 12 et 14 dans lequel Z représente un groupe -CONH$_2$

10. Peptide suivant la revendication 1, répondant à la formule

Ala-His-B$^\beta$Nal-Ala-Trp-DPhe-Lys-NH$_2$,
Ala-His-$D^\beta$Nal-Ala-Trp-DPhe-Ala-Lys-NH$_2$,
Ala-His-$D^\beta$Nal-Ala-Trp-DPhe-Ala-Ala-Lys-NH$_2$,
Lys-His-$D^\beta$Nal-Ala-Trp-DPhe-$A_6$-NH$_2$ ou
Glu-His-$D^\beta$Nal-Ala-Trp-DPhe-$A_6$-NH$_2$.

11. Peptide suivant la revendication 2, 5, 7 ou 14, dans lequel $A_6$ représente G.

12. Peptide suivant la revendication 1, répondant à la formule His-Ala-$D^\beta$Nal-Ala-Trp-DPhe-$A_6$-Z.

13. Peptide suivant la revendication 12, répondant à la formule His-Ala-$D^\beta$Nal-Ala-Trp-DPhe-Lys-NH$_2$.

14. Peptide suivant la revendication 1, répondant à la formule Ala-$D^\beta$Nal-Ala-Trp-DPhe-$A_6$-Z.

15. Peptide suivant la revendication 14, répondant à la formule Ala-$D^\beta$Nal-Ala-Trp-DPhe-Lys-NH$_2$.

16. Méthode non thérapeutique pour provoquer la libération d'hormone de croissance et accroître les taux de cette hormone chez un animal autre que l'homme, comprenant l'administration à l'animal d'une quantité efficace d'au moins un des peptides suivant la revendication 1, 4, 7, 10 ou 12.

17. Procédé suivant la revendication 16, dans lequel l'animal autre que l'homme est un mammifère.

18. Composition pharmaceutique destinée à provoquer la libération d'hormone de croissance et à augmenter les taux de cette hormone chez des animaux, comprenant au moins un des peptides suivant la revendication 1, 4, 7, 10 ou 12 et un support ou diluant pharmaceutiquement acceptable.

19. Peptide suivant la revendication 2, dans lequel $A_0$ représente n'importe quel amino-acide naturel.

20. Association synergique du peptide suivant la revendication 1 et d'un second composé, dans laquelle ledit second composé est un composé qui agit comme agoniste au niveau du récepteur d'hormone de libération de l'hormone de croissance ou qui inhibe la libération de somatostatine, apte à provoquer la libération et l'augmentation des taux sanguins d'hormone de croissance.

**21.** Composition pharmaceutique suivant la revendication 18, qui comprend en outre un second composé qui agit comme agoniste au niveau du récepteur d'hormone de libération de l'hormone de croissance ou qui inhibe la libération de somatostatine.

**22.** Utilisation d'un composé de formule :

$A_1$-$A_2$-$A_3$-Trp-$A_5$-$A_5$-Z

dans laquelle $A_1$ représente un groupe His, 3(NMe)His, His-Ala, Ala, Tyr, $A_0$-His ou $A_0$-3(NMe)His, dans lequel $A_0$ représente n'importe quel L-amino-acide naturel, Met(O), DOPA, Abu ou des peptides de formule L-$A_0$, dans laquelle L représente H, DOPA, Lys, Phe, Tyr, Cys, Tyr-DAla-Phe-Gly, Tyr-DAla-Gly-Phe, Tyr-Ala-Gly-Thr ou Tyr-DAla-Phe-Sar ;

$A_2$ représente $D^\beta Nal$ ;

$A_3$ représente Ala, Gly ou Ser ;

$A_5$ représente DPhe, $D/L^\beta$(Me)Phe ou (NMe)DPhe ;

$A_5$ représente B-G ou G, dans la production d'un médicament destiné à provoquer la libération d'hormone de croissance dans le sang et à élever le taux de cette hormone dans le sang.

**23.** Procédé pour la préparation d'un composé de formule

$A_1$-$A_2$-$A_3$-Trp-$A_5$-$A_5$-Z

dans laquelle $A_1$ représente His, 3(NMe)His, His-Ala, Ala, Tyr, $A_0$-His ou $A_0$-3(NMe)His, dans lequel $A_0$ représente n'importe quel L-amino-acide naturel, Met(O), DOPA, Abu ou des peptides de formules L-$A_0$, dans laquelle L représente H, DOPA, Lys, Phe, Tyr, Cys, Tyr-DAla-Phe-Gly, Tyr-DAla-Gly-Phe, Tyr-Ala-Gly-Thr ou Tyr-DAla-Phe-Sar ;

$A_2$ représente $D^\beta Nal$ ;

$A_3$ représente Ala, Gly ou Ser ;

$A_5$ représente DPhe, $D/L^\beta$(Me)Phe ou (NMe)DPhe ;

$A_5$ représente B-G ou G, qui comprend le couplage d'amino-acides ou de dérivés d'amino-acides pour former le composé.

**24.** Procédé de préparation du peptide suivant la revendication 1, qui comprend une réaction de condensation de fragments peptidiques pour former le peptide.